Europäisches Patentamt

European Patent Office

Office européen des brevets

(11) Veröffentlichungsnummer: **0 073 308**
A1

# EUROPÄISCHE PATENTANMELDUNG

(21) Anmeldenummer: 82105314.7

(22) Anmeldetag: 17.06.82

(51) Int. Cl.³: **A 61 M 23/00, A 61 M 25/00**

(30) Priorität: 17.08.81 DE 8123912 U

(43) Veröffentlichungstag der Anmeldung: 09.03.83
Patentblatt 83/10

(84) Benannte Vertragsstaaten: **AT BE CH DE FR GB IT LI LU NL SE**

(71) Anmelder: **INTERMEDICAT GMBH, Gerliswilstrasse 43, CH-6020 Emmenbrücke (CH)**

(72) Erfinder: **Herlitze, Gerhard, Binsdorfer Strasse 4, CH-3507 Baunatal 7 (CH)**

(74) Vertreter: **von Kreisler, Alek, Dipl.-Chem. et al, Deichmannhaus am Hauptbahnhof, D-5000 Köln 1 (DE)**

(54) **Sonde aus Kunststoff.**

(57) Die Erfindung betrifft eine Sonde (2) aus Kunststoff mit hohem Röntgenkontrastmittelanteil und beträchtlicher Zugfestigkeit. Sie enthält mindestens eine in den Kunststoff eingebettete reißfeste Seele (3). Bei Vorhandensein nur einer Seele (3′) ist diese vorzugsweise konzentrisch innerhalb der Sonde (2) angeordnet.

EP 0 073 308 A1

0073308

## Sonde aus Kunststoff

Die Erfindung bezieht sich auf eine Sonde aus Kunststoff zur Ausfüllung des Innenraumes eines Katheterschlauches, die den Rückfluß von Flüssigkeit aus dem Katheterschlauch hemmt und den ,Katheterschlauch zur Erleichterung seiner Einführung in ein Blutgefäß versteift.

Langzeittherapien von Infusionslösungen werden vorzugsweise über Venenkatheter eingeleitet. Es sind verschiedene Ausführungsformen von Venenkathetern bekannt, die sich durch Länge, Durchmesser und Anwendungstechnik unterscheiden. Meistens werden Venenkatheter ohne zur Hilfenahme des Röntgenschirmes gelegt, wobei allerdings eine nachträgliche Lagekontrolle durchgeführt wird.

In manchen Fällen, z.B. bei der Verlegung eines zum Herzen führenden Katheters, muß der Vorschub des Katheterschlauches jedoch mit dem Röntgenschirm überwacht werden. Zur Darstellung des Katheterschlauches

- 2 -

am Röntgenschirm wird bisher entweder der Katheterschlauch selbst in oder an seiner Wand mit Röntgenkontraststreifen versehen, bzw. er wird vollpigmentiert
hergestellt oder es wird die aus einem röntgenkontrastmittelhaltigen Kunstoff bestehende, den Innenraum des
Katheterschlauches ausfüllende Sonde zur Verbesserung
der Darstellung am Röntgenschirm benutzt. Die den
verschiedenen Arten von Kunststoffen beigemengten
Röntgenkontrastmittel verringern die Festigkeit des
Ausgangsmaterials und beeinträchtigen je nach
prozentualem Anteil die Zugfestigkeit erheblich. Dies
kann zu Abrissen der Sonde führen, die für den Patienten
gefährlich sind.

Der Erfindung liegt die Aufgabe zugrunde, die Zugfestigkeit einer Sonde mit hohem Röntgenkontrastmittelanteil zu verbessern.

Diese Aufgabe wird bei einer Sonde aus Kunststoff
gelöst durch mindestens eine in den Kunststoff eingebettete reißfeste Seele.

Diese Sonde kann nach dem bekannten Extrusionsverfahren für Kabelummantelung einfach hergestellt werden.
Ihre Einlage vergrößert ihre Zugfestigkeit, so daß sie
bei erhöhtem Röntgenkontrastmittelgehalt des Sondenwerkstoffes nicht abreißen kann. Außerdem kann die
Seele selbst aus röntgenfähigem Werkstoff bestehen und
die röntgenologische Sichtbarkeit des Katheterschlauches

- 3 -

mit Sonde  zusätzlich steigern.

In vorteilhafter Ausgestaltung der Erfindung ist die Seele konzentrisch innerhalb der Sonde angeordnet, so daß sie allseitig von dem Werkstoff der Sonde ummantelt ist.

Die Seele kann aus rostfreiem Draht, vorzugsweise Stahldraht,bestehen. Alternativ kann sie aus reißfesten gesponnenen Fäden, wie Silberfäden oder reißfestem Zwirn hergestellt sein.

In weiterer Ausgestaltung der Erfindung kann die Seele aus einem Monofil eines vom Werkstoff der Sonde abweichenden Werkstoffes bestehen.

In der Zeichnung ist ein Ausführungsbeispiel der Erfindung schematisch dargestellt. Es zeigt:

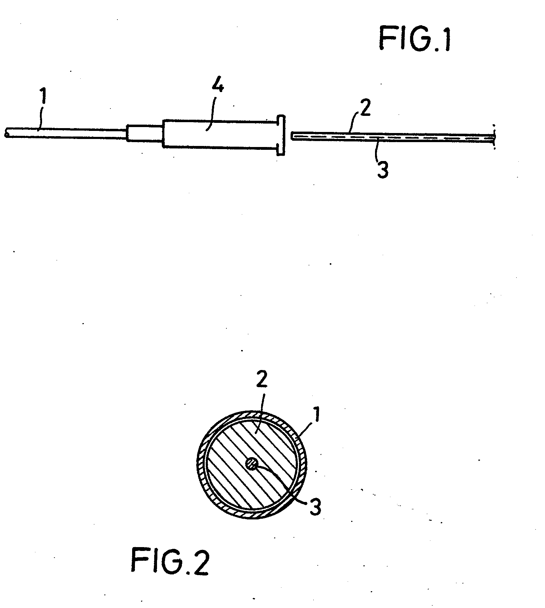

Figur 1    ein Stück einer Sonde vor Einführung in den Katheteransatz eines Katheterschlauches, und

Figur 2    einen Querschnitt einer in einem Katheterschlauch steckenden Sonde in vergrößertem Maßstab.

Eine den Hohlraum eines Katheterschlauches 1 ausfüllende und den Blutrückfluß hemmende Sonde 2 aus röntgenkontrastmittelhaltigem Kunststoff ist mit einer konzentrisch in den Kunststoff eingebetteten

- 4 -

reißfesten Seele 3 versehen. Die Seele 3 kann aus rostfreiem Stahldraht bestehen. Zusätzlich zu dem in dem Werkstoff der Sonde 2 enthaltenen hohen Röntgenkontrastmittelanteil verbessert die Seele 3 die Darstellung des Katheterschlauches 1 am Röntgenschirm. Ferner erhöht die Seele 3 die Zugfestigkeit der Sonde 2 und verhindert ihr Abreißen.

Am körperfernen Ende der Sonde befindet sich ein nicht gezeichnetes Griffstück, das mit dem Katheteransatz 4 des Katheterschlauches 1 lösbar zusammensteckbar ist, so daß der Katheterschlauch 1 und die Sonde 2 bei der Einführung in das Blutgefäß als Einheit gehandhabt werden können.

- 5 -

<u>A n s p r ü c h e</u>

1. Sonde aus Kunststoff zur Ausfüllung des Innenraumes eines Katheterschlauches, g e k e n n - z e i c h n e t   d u r c h   mindestens eine in den Kunststoff eingebettete reißfeste Seele (3).

2. Sonde nach Anspruch 1, dadurch gekennzeichnet, daß die Seele (3) konzentrisch innerhalb der Sonde (2) angeordnet ist.

3. Sonde nach Anspruch 1 oder 2, dadurch gekennzeichnet, daß die Seele (3) aus rostfreiem Draht besteht.

4. Sonde nach Anspruch 1 oder 2, dadurch gekennzeichnet, daß die Seele (3) aus reißfesten gesponnenen Fäden besteht.

5. Sonde nach Anspruch 1 oder 2, dadurch gekennzeichnet, daß die Seele (3) aus einem Monofil eines vom Werkstoff der Sonde (2) abweichenden Werkstoffes besteht.

6. Sonde nach einem der Ansprüche 1 bis 5, gekennzeichnet durch hohen Röntgenkontrastmittelanteil ihres Werkstoffes.

0073308

FIG.1

1  4  2

3

FIG.2

2

1

3

Europäisches
Patentamt

**EUROPÄISCHER RECHERCHENBERICHT**

0073308

Nummer der Anmeldung

EP 82 10 5314

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | A 61 M 23/00 |
| X,Y | US-A-2 268 321 (V.J. FLYNN) * Anspruch 1; Seite 1, rechte Spalte, Zeilen 31-46; Seite 2, linke Spalte, Zeilen 32-36; Seite 3, rechte Spalte, Zeilen 47-70; Seite 3, linke Spalte, Zeilen 48-55 * | 1-6 | A 61 M 25/00 |
| | --- | | |
| Y | US-A-3 572 333 (F.H. HUBERT) * Ansprüche 1, 2, 4; Spalte 1, Zeilen 47-52; Spalte 2, Zeilen 44-52; Spalte 3, Zeilen 25-29 * | 1-6 | |
| | --- | | |
| Y | GB-A-1 435 797 (A/S SURGIMED) * Anspruch 1; Seite 1, Zeilen 11-28; Seite 2, Zeilen 5, 6 * | 1-3,6 | |
| | --- | | RECHERCHIERTE SACHGEBIETE (Int. Cl. ³) |
| A | EP-A-0 014 424 (TORAY MONOFILAMENT CO. LTD.) * Ansprüche 1-3; Seite 6, Zeilen 32-37; Seite 7, Zeilen 1, 7, 8; Figuren 1A, 1B, 6 * | 1-3,6 | A 61 M 23/00 A 61 M 25/00 |
| | --- | | |
| A | DE-A-2 542 241 (G.S. LINDER et al.) * Ansprüche 1-4; Figur 3 * | 1,2 | |
| | --- | | |
| A | DE-B-1 491 628 (T.H.O. ALMEN) * Spalte 3, Zeilen 51-60; Spalte 7, Zeilen 34-40; Figur 5 * | 6 | |
| | ----- | | |

Der vorliegende Recherchenbericht wurde für alle Patentansprüche erstellt.

| Recherchenort | Abschlußdatum der Recherche | Prüfer |
|---|---|---|
| BERLIN | 25-10-1982 | CLOT P.F.J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503. 03.82